# EUROPÄISCHE PATENTSCHRIFT

(11) **EP 2 086 410 B1**
(45) Veröffentlichungstag und Bekanntmachung des Hinweises auf die Patenterteilung: **02.05.2012**
(21) Anmeldenummer: 07821802.1
(22) Anmeldetag: 24.10.2007
(51) Int. Cl.: A61B 5/11, A61B 5/103

(54) **VORRICHTUNG ZUM BEFESTIGEN ZUMINDEST EINES FASERBANDS AUF EINEM KÖRPERTEIL SOWIE EIN VERFAHREN ZU DESSEN HERSTELLUNG**
DEVICE FOR FIXING AT LEAST ONE FIBROUS STRIP TO A BODY PART, AND METHOD FOR THE PRODUCTION OF SAID STRIP
DISPOSITIF DE FIXATION D'AU MOINS UNE BANDE DE FIBRES SUR UNE PARTIE DU CORPS ET PROCÉDÉ DE FABRICATION DE LA BANDE DE FIBRES

(30) Priorität: 02.11.2006 DE 102006051742; 15.06.2007 DE 102007027722
(43) Veröffentlichungstag der Anmeldung: 12.08.2009
(60) Teilanmeldung: 12001407.1
(73) Patentinhaber: SIEMENS AKTIENGESELLSCHAFT, 80333 München (DE)
(72) Erfinder: GOLDBECK, Dirk David, 81927 München (DE); HAPPEL, Tobias, 14089 Berlin (DE); SOMMER, Daniel, 89075 Ulm (DE); L'HENORET, Benjamin, 31000 Toulouse (FR); NOWSCH, Helmut, 93059 Regensburg (DE)
(86) Internationale Anmeldenummer: PCT/EP2007/061439
(87) Internationale Veröffentlichungsnummer: WO 2008/052926

(56) Entgegenhaltungen:
- WO-A-03/060449
- WO-A-2007/110300
- US-A- 5 242 440

## Beschreibung

Die Erfindung beschäftigt sich mit einer Vorrichtung zum Befestigen zumindest eines Faserbands auf einem Körperteil, sowie mit einem Verfahren zum Herstellen einer Vorrichtung zum Befestigen von zwei Faserbändern in paralleler Anordnung auf einem Körperteil.

Zur Vermessung von Biegewinkeln, bspw. durch Biegen eines Fingergelenks, ist bekannt, faseroptische Biegesensoren einzusetzen, siehe [1]. Der faseroptische Biegesensor weist dabei eine oder mehrere sensitive Zonen auf, die derart behandelt sind, dass aufgrund der Biegung des faseroptischen Biegesensors eine Lichtdämpfung in Abhängigkeit eines Biegewinkels sich ändert.

Diese Technologie wird seit längerer Zeit auch zur Vermessung von Verbiegungen und Verdrehungen von Körperteilen, wie z.B. dem Verbiegen eines Fingergelenks, bei Mensch und Tier eingesetzt.

In einem Dokument US 5,242,440 wird ein Schalt-Apparat vorgestellt, der eine Ausgabeeinheit, eine Stromversorgungseinheit zum Bereitstellen von Strom an die Ausgabeeinheit und einen am Finger angerachten Schalter aufweist, wobei der Schalter zwischen der Ausgabeeinheit und der Stromversorgungseinheit bereitgestellt ist.

Ein Dokument WO 2007/110300A1 beschreibt einen Gerät, einen Sensor, ein Sensorelement und eine Methode um sowohl das Profil der Wirbelsäule als auch Änderungen im Profil der Wirbelsäule zu messen.

Hierbei stellt sich die Aufgabe, eine Vorrichtung zur Befestigung von zumindest einem faseroptischen Biegesensor derart vorzusehen, dass dieser trotz einer Änderung einer zu vermessenden Körperoberfläche zuverlässige Messergebnisse liefert.

Diese Aufgabe wird durch die unabhängigen Ansprüche gelöst. Weiterbildungen sind den abhängigen Ansprüchen zu entnehmen.

Die Erfindung betrifft eine Vorrichtung zum Befestigen zumindest eines Faserbands auf einem Körperteil, wobei das Faserband zumindest eine Faser umfasst, die als faseroptischer Biegesensor ausgestaltet ist, wobei die Vorrichtung zumindest eine Befestigungseinheit aufweist und die jeweilige Befestigungseinheit (a) ein Befestigungsmittel zum Anbringen eines Teils des Faserbands auf dem Körperteil und (b) zumindest eine Befestigungsschlaufe zum Führen des Faserbands in einer Achse eines dreidimensionalen Raums, wobei die Befestigungsschlaufe auf dem Körperteil befestigbar ist, umfasst.

Diese Vorrichtung ermöglicht zuverlässige Messergebnisse, da die Faser auf einem sich bewegenden Körperteil sowohl den Bewegungen des Körperteils folgenden als auch durch die Bewegungen nicht derart verrutschen kann, dass eine Messung der Bewegungen durch die Verrutschung fehlerhaft wird. Insbesondere das Befestigungsmittel sorgt dafür, dass die Faser dieselbe Position wieder einnimmt, nachdem sich das Körperteil wieder in seine ursprüngliche Position bewegt hat.

Ist die zumindest eine Befestigungsschlaufe und/oder das Befestigungsmittel der zumindest einen Befestigungseinheit auf einem dehnbaren Befestigungsträger, insbesondere einem Pflastermaterial, aufgebracht ist, wobei der dehnbare Befestigungsträger mit dem Körperteil durch einen Klebstoff befestigbar ist, so kann die Vorrichtung in einfacher Art und Weise am Körperteil befestigt und wieder vom Körperteil entfernt werden, wobei eine präzise Befestigung mit einfacher Korrekturmöglichkeit gegeben ist. Ferner zeigt diese Weiterbildung den Vorteil, dass das dehnbare Befestigungsmaterial auch Längenänderungen des Körperteils durch z.B. Beugen und Strecken des Rückens folgen kann, ohne dass sich die Vorrichtung derart verschiebt, dass die Messergebnisse unzuverlässig werden.

In einer Weiterbildung umfasst die Vorrichtung eine einzige Befestigungseinheit mit einem einzigen Befestigungsmittel und zwei oder mehreren Befestigungsschlaufen, das Körperteil ist ein Rücken, das Befestigungsmittel ist oberhalb der Befestigungsschlaufen, insbesondere im oberen Bereich des Rückens, angeordnet, und die Befestigungsschlaufen sind untereinander am Rücken angeordnet.

Diese Weiterbildung stellt eine kostengünstige Realisierung der Vorrichtung dar.

In einer alternativen Weiterbildung umfasst die Vorrichtung zwei oder mehrere Befestigungseinheiten, wobei jede der Befestigungseinheiten ein einziges Befestigungsmittel, zumindest eine Befestigungsschlaufe und ein Faserband mit zumindest einer Faser umfasst, und wobei die Befestigungseinheiten untereinander am Rücken angeordnet sind.

Hierdurch ist die Vorrichtung derart ausgebildet, dass sie großen Änderungen des Rückens gut folgen kann, da die die Vorrichtung mit mehreren Befestigungseinheiten ausgestaltet ist, die bspw. nur eine kurze örtliche Ausdehnung, z.B. 7 cm, aufweisen.

Wird hierbei das Befestigungsmittel einer ersten Befestigungseinheit oberhalb einem unterem Ende des Faserbands einer, oberhalb der ersten Befestigungseinheit angebrachten, zweiten Befestigungseinheit angeordnet, so ist ein kontinuierliches Messen mittels der jeweiligen Befestigungseinheiten gewährleistet.

In einer optionalen Weiterbildung umfasst jede Befestigungseinheit eine sensitive Zone für zumindest eine Achse eines dreidimensionalen Raums umfasst. Hiermit kann mit einer sensitiven Zone eine Bewegungsrichtung des Körperteils vermessen werden. Mit mehreren sensitiven Zonen unterschiedlicher Achsen sind ferner mehrere unterschiedliche Bewegungsrichtungen der Bewegung messbar.

In einer vorzugsweisen Weiterbildung der Erfindung sind die Befestigungsschlaufen der zumindest einen Befestigungseinheit parallel zu einer Wirbelsäule des Rückens oder auf der Wirbelsäule des Rückens anordenbar. Hiermit kann den Bewegungen der Wirbelsäule durch die Faser bzw. Fasern gut gefolgt werden, womit eine hohe Messgenauigkeit bei einem Vermessen einer Bewegung der Wirbelsäule erzielt wird.

Vorzugsweise umfasst die Vorrichtung zwei Befestigungseinheiten, die zwei Befestigungseinheiten sind in Längsrichtung parallel nebeneinander angeordnet, insbesondere sind die eine der zwei Befestigungseinheiten links und die andere der zwei Befestigungseinheiten rechts von einer Wirbelsäule eines Patienten anordenbar. Durch Verwendung von zwei in Längsrichtung parallel nebeneinander verlaufender Befestigungseinheiten ist eine Steigerung der Messgenauigkeit erzielbar, da neben einer Dopplung von Messergebnissen in bestimmten Bewegungsrichtungen auch Verdrehungen erfassbar sind.

In einer optionalen Weiterbildung hierzu weist die jeweilige Befestigungseinheit eine einzige Befestigungsschlaufe auf, wobei die Befestigungsschlaufe aus einem in Längsrichtung der Vorrichtung ausgedehnten schlauchförmigen Tubus aus einem dehnbaren Material mit einem an der Außenseite des Tubus versehenen Klebematerials gebildet ist, die derart gebildeten Befestigungsschlaufen der beiden Befestigungseinheiten auf dem dehnbaren Befestigungsträger parallel in Längsrichtung der Vorrichtung angeordnet sind, eine weiteres in Längsrichtung der Vorrichtung ausgedehntes dehnbares Material mit seiner mit Klebematerial versehnen Unterseite auf den Befestigungsschlaufen und seitlich der Befestigungsschlaufen mit dem dehnbaren Befestigungsträger verbunden ist. Mit dieser optionalen Weiterbildung kann die Vorrichtung in besonders einfacher, insbesondere industriell fertigbarer, Weise hergestellt werden.

In einer alternativen Weiterbildung sind die Befestigungsschlaufen der jeweiligen Befestigungseinheit aus jeweils zwei sich im Bereich des Faserbands überkreuzenden Dehnungsfäden ausgebildet. Die Verwendung von sich überkreuzenden Dehnungsfäden zum Fixieren der Faserbänder als Befestigungsschlaufen zeigt den Vorteil, dass ein derartiger Aufbau sowohl leicht ist, da nur die Dehnungsfäden ein geringes gewicht aufweisen, als auch, dass diese Dehnungsfäden ein sicheres Fixieren der Faserbänder auch bei größeren Längenänderungen des Befestigungsträgers gewährleisten. Ferner können bei Anbringen der Vorrichtung auf das Körperteil die Dehnungsfäden bereits derart gedehnt, d.h. vorgespannt, werden, dass bei einer Verringerung der Körperoberfläche diese sich zusammenziehen und somit eine gute Fixierung der Faserbänder ermöglichen. Durch die gute Fixierung wird ein gutes Messergebnis erzielt.

Werden die beide Befestigungseinheiten auf dem einen dehnbaren Befestigungsträger angebracht, so kann die Vorrichtung Längenänderungen der Körperoberfläche des Körperteils gut folgen. Ferner zeigt sich eine Steigerung der Messgenauigkeit, falls der dehnbare Befestigungsträger lediglich in Längsrichtung der Vorrichtung dehnbar ist, da dadurch Messfehler durch Dehnungen in Querrichtung der Vorrichtung vermieden werden.

Vorzugsweise sind auf links und/oder rechts der jeweiligen Faserbänder verlaufende Bereiche des dehnbaren Befestigungsträgers ein weiteres dehnbares Material mit seiner mit Klebematerial versehnen Unterseite aufgebracht. Hiermit lässt sich sowohl eine Stabilität der Vorrichtung erhöhen, als auch kann dadurch das Faserband besser gegen seitliches Verrutschen gesichert werden. Insbesondere sind die Dehnungsfäden mit dem weiteren dehnbaren Material verbunden sind.

Vorzugsweise ist im Bereich der jeweiligen Befestigungsschlaufe unter eine zum Körper zeigende Seite des jeweiligen Faserbands ein zum Gleiten des Faserbands förderndes Gleitmaterial angeordnet. Hierdurch wird die Messgenauigkeit erhöht, da Messfehler durch ein Verkanten des Faserbands bei Bewegungen des Körperteils reduziert oder vermieden wird. Insbesondere ist als Gleitmaterial ein beidseitig beschichtetes Teflonband geeignet, da dies durch seine Beweglichkeit den Bewegungen des Rückens gut folgen kann und somit keine Messfehler hervorruft.

Die Erfindung betrifft auch ein Verfahren zum Herstellen einer Vorrichtung zum Befestigen von zwei Faserbändern in paralleler Anordnung mittels eines Ausgangsmaterials umfassend ein Trägermaterial und ein auf einer Unterseite des Trägermaterials angeordnetes Klebematerial auf einem Körperteil, bei dem folgende Schritte durchgeführt werden:
- aus einem werden zwei schlauchförmige Tubi derart geformt, dass sich das Klebematerial auf der Außenseite des jeweiligen Tubus befindet.
- die zwei schlauchförmigen Tubi werden in Längsrichtung in paralleler Anordnung auf die Oberseite des durch das Ausgangsmaterial gebildeten Befestigungsträgers aufgeklebt, wobei der Befestigungsträger zum Anbringen der Vorrichtung auf ein Körperteil ausgestaltet ist.
- In Längsrichtung wird über den mit den zwei schlauchförmigen Tubi versehenen Befestigungsträger ein weiteres dehnbares Material in Form des Ausgangsmaterials mit Hilfe des Klebematerials, aufgebracht.

Mit diesem verfahren kann die Vorrichtung in besonders einfacher, insbesondere industriell fertigbarer, Weise hergestellt werden.

Die Erfindung und ihre Weiterbildungen werden anhand von Zeichnungen näher erläutert. Im Einzelnen zeigen:
- Figur 1: Faserband mit drei einzelnen Fasern zur Messung einer Biegung in unterschiedlichen Achsen eines dreidimensionalen Raums;
- Figur 2: Faserband mit mehrerer Fasern, wobei sensitive Zonen der Fasern in einer Achse des dreidimensionalen Raums, aber an unterschiedlichen Position im Raum, angeordnet sind;
- Figur 3: Ein erstes Ausführungsbeispiel mit einer Befestigungseinheit;
- Figur 4: Ein zweites Ausführungsbeispiel mit mehreren Befestigungseinheiten;
- Figur 5: Ein dehnbares Trägermaterial mit einem Klebematerial auf seiner Unterseite;
- Figur 6: Eine weitere Ausführungsform mit zwei parallel verlaufenden Befestigungseinheiten in einem Querschnitt;
- Figur 7: Die weitere Ausführungsform mit zwei parallel verlaufenden Befestigungseinheiten in einer Draufsicht;
- Figur 8: Ablaufdiagramm zur Herstellung einer Vorrichtung zumindest zwei parallel verlaufender Befestigungseinheiten;
- Figur 9: Ein letztes Ausführungsbeispiel der Erfindung mit zwei parallel verlaufenden Faserbändern, die jeweils mit sich überkreuzenden Dehnfäden als Befestigungsschlaufen fixiert werden;
- Figur 10: Querschnitt des letztes Ausführungsbeispiels.

Elemente mit gleicher Funktion und Wirkungsweise sind in den Figuren mit denselben Bezugszeichen versehen.

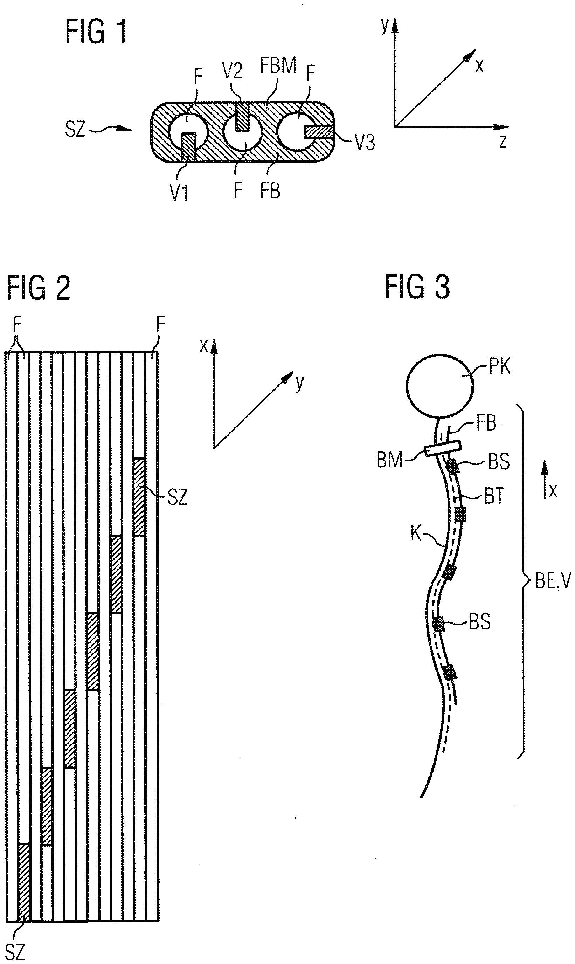

Faseroptische Biegesensoren sind bspw. aus [1] bekannt. Dabei wird eine Oberfläche einer Faser F an einer sensitiven Zone SZ derart verändert, dass ein in die Faser eingekoppelter Lichtstrahl durch Verbiegen der Faser an der sensitiven Zone gedämpft wird. Je nach Biegung der Faser ändert sich die Dämpfung. In Figur 1 ist ein drei Fasern umfassendes Faserband FB zu sehen, wobei die drei Fasern durch einen Faserbandmantel FBM mechanisch miteinander verbunden sind. Jede Faser weist eine mechanische Veränderung V1, V2, V3 an einer jeweiligen Faseroberfläche auf. Durch diese mechanischen Veränderungen, z.B. realisiert durch Herausnahme von Fasermaterial und Verfüllung mit einem vom Fasermaterial verschiedenen Material, entsteht die jeweilige sensitive Zone mit einer Biegeempfindlichkeit in der Achse (x, y, z) eines dreidimensionalen Raums.

In Figur 2 sind eine Vielzahl von Fasern F in Längsrichtung nebeneinander als Faserband FB angeordnet. Die Fasern weisen jeweils eine mechanische Verletzung auf, wobei jeweilige mechanische Verletzung an unterschiedlichen Positionen in einer Achse x des dreidimensionalen Raums angeordnet sind, wodurch eine Biegung an den unterschiedlichen Positionen des Faserbanda erfassbar sind. Beispielsweise sind alle mechanischen Verletzungen, die die jeweilige sensitive Zone SZ repräsentieren, auf der Oberseite der Faser angebracht, so dass eine jeweilige Biegung in einer weiteren Achse y des dreidimensionalen Raums ermittelbar sind. Das hier vorgestellte Faserband ist eine mögliche Realisierungsvariante, wobei die vorliegende Erfindung auf beliebige Anordnungen der sensitiven Zonen bzw. Verletzungen bei im Faserband parallel verlaufenden Fasern anwendbar ist.

Figur 3 zeigt ein erstes Ausführungsbeispiel der Erfindung. Hierbei ist schematisch eine Person mit einem Kopf PK und einem Rücken zu sehen, wobei der Rücken das zu vermessende Körperteil K repräsentiert. Hierbei soll insbesondere eine Form und eine Verformung der Wirbelsäule über der Zeit gemessen werden. Ferner ist in Figur 3 eine Befestigungseinheit BE abgebildet, die ein Befestigungsmittel BM, mehrere Befestigungsschlaufen BS und einen dehnbaren Befestigungsträger BT umfasst. Zur Messung wird bspw. das Faserband FB gemäß Figur 2 eingesetzt.

Der Befestigungsträger BT, mit einer gestrichelten Linie in Figur 3 eingezeichnet, besteht aus einem dehnbaren Material, welches auf seiner Unterseite, die mit dem Körperteil verbunden wird, Klebematerial, d.h. Klebestoff aufweist. Dieser Befestigungsträger ist bspw. ein Pflastermaterial CureTape® vertrieben von einer Firma PhysioTape B.V., Niederlande, siehe auch http://www.fysiotape.nl/DE/Products/DE2938493234.doc/

Der Befestigungsträger wird zum Anbringen der Vorrichtung auf den Rücken auf diesen aufgeklebt. Der Befestigungsträger kann, wie in Figur 3 beispielhaft wiedergegeben, durchgehend auf dem Rücken des Patienten aufgebracht werden. Alternativ kann der Befestigungsträger abschnittsweise an dem Rücken angebracht werden, wobei es die Befestigungsschlaufen und das Befestigungsmittel mit dem Rücken, verbindet.

Bei der Befestigungseinheit BE gemäß der beispielhaften Ausführung nach Figur 3 ist das Befestigungsmittel BM am oberen Ende der Wirbelsäule angeordnet, wobei das Befestigungsmittel die Aufgabe hat, das Faserband FB an einer Stelle der Wirbelsäule gegen Verschiebung zu fixieren. Das Befestigungsmittel ist bspw. als Klebeband oder Klammer ausgeführt. In dem vorliegenden Beispiel wird das Befestigungsmittel mit dem Befestigungsträger am Rücken angebracht. In einer alternativen Ausführung wird das Befestigungsmittel direkt auf dem Rücken befestigt. Das Befestigungsmittel BM kann auch am unten Ende des Rückens das Faserband fixieren, so dass das den Rücken nach oben zeigende Ende des Faserbands in den Befestigungsschlaufen gleiten kann. Im Allgemeinen kann das Befestigungsmittel das Faserband an jeder beliebigen Position am Rücken fixieren, z.B. die Mitte des Faserbands in etwa der Mitte des Rückens.

Unterhalb des Befestigungsmittels gemäß Figur 3 sind mehrere Befestigungsschlaufen BS angeordnet, die das Faserband FB aufnehmen und ermöglichen, dass das Faserband jeweils in eine Achse des dreidimensionalen Raums gleiten kann. Durch Beugen und Strecken des Rückens verändert sich die Oberfläche des Rückens. Die Befestigungsschlaufen bewirken dabei, dass das relativ starre Faserband dem Oberflächenverlauf der Wirbelsäule trotz Beugung bzw. Streckung des Rückens folgt. Die Befestigungsschlaufen sind bspw. aus einem Kunststoffring oder einem Gummiring geformt und werden mittels des Befestigungsträgers BT an der Wirbelsäule befestigt. In Figur 3 umfasst eine Vorrichtung V zum Befestigen zumindest eines Faserbands genau eine Befestigungseinheit BE. Im Allgemeinen kann das Befestigungsmittel an jeder Position am Rücken und an dem Faserband angebracht werden, so dass je nach Anordnung die Befestigungsschlaufen oberhalb, unterhalb oder sowohl oberhalb und als auch unterhalb des Befestigungsmittel angeordnet sein können. Dies gilt auch für die weiteren Ausführungsformen.

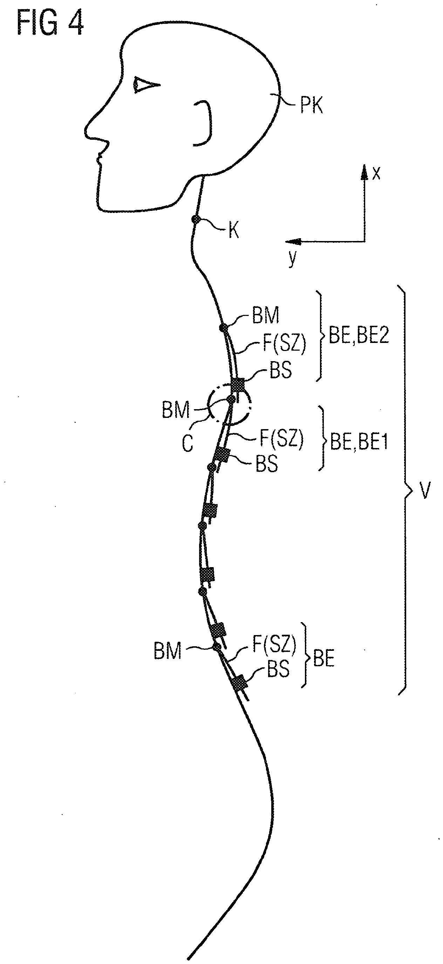

In Figur 4 ist eine weitere Ausführungsform der Erfindung abgebildet. Hierbei umfasst die Vorrichtung V mehrere Befestigungseinheiten BE zur Vermessung einer Form des Körperteils K, d.h. des Rückens. Jede Befestigungseinheit umfasst dabei ein Faserband mit bspw. einer sensitiven Zone, die mittels zumindest einer Faser ausgebildet ist. Ferner weist jede Befestigungseinheit ein Befestigungsmittel BM und zumindest eine Befestigungsschlaufe BS auf. Sowohl das Befestigungsmittel als auch die Befestigungsschlaufen sind derart ausgebildet, dass diese direkt auf den Rücken bzw. die Wirbelsäule anbringbar sind, z.B. mittels eines Klebematerials oder mittels eines ansaugenden Materials. In Figur 4 wird das jeweilige Faserband mit dem Befestigungsmittel oberhalb der Befestigungsschlaufe am Rücken fixiert. Beispielsweise wird an jedem dritten Wirbel eine Befestigungseinheit angebracht. In Figur 4 sind die erste und zweite Befestigungseinheit BE1, BE2 zu sehen. Um ein kontinuierliches Messen zu ermöglichen sind die erste und zweite Befestigungseinheit BE1, BE2 der Vorrichtung V derart angeordnet, dass das freie, d.h. nicht durch das Befestigungsmittel fixierte Ende, des Faserbands der zweiten Befestigungseinheit über das Befestigungsmittel eines Faserbands der benachbarten ersten Befestigungseinheit BE1 hinausragt. Dies ist in Figur 4 in einem gestrichelten Kreis mit einem Bezugszeichen C zu sehen.

Die folgenden Ausführungsbeispiele betreffen eine Vorrichtung V mit mehreren Befestigungseinheiten BE, wobei jeweils zwei der Befestigungseinheiten BE in Längsrichtung parallel nebeneinander angeordnet sind, insbesondere die eine der zwei Befestigungseinheiten BE links und die andere der zwei Befestigungseinheiten BE rechts von einer Wirbelsäule eines Patienten anordenbar sind.

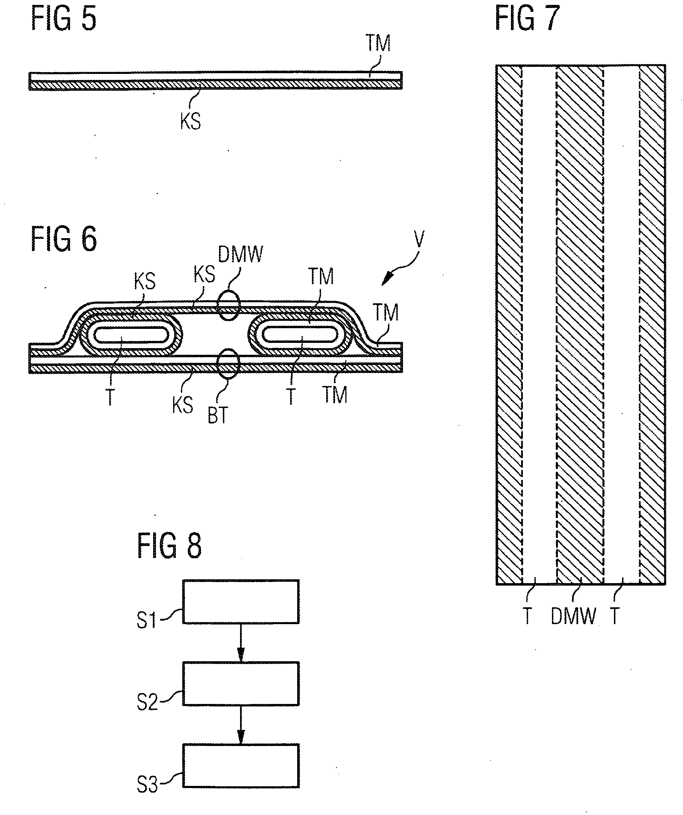

In einem Ausführungsbeispiel gemäß den Figuren 5 bis 7 wird die Vorrichtung V vorgestellt, die in kostengünstiger Weise herstellbar ist. Als Ausgangsmaterial dient ein dehnbares Trägermaterial TM mit einem Klebematerial KS auf seiner Unterseite, siehe Figur 5. Beispielsweise kann ein handelsübliches Pflastermaterial, wenn möglich auf Spulen aufgewickelt, hierfür eingesetzt werden, wie z.B. CureTape®. Das Herstellverfahren läuft dabei in folgenden Schritten gemäß Figur 8 ab:
Schritt S1:
   Aus dem Ausgangsmaterial werden zwei schlauchförmige Tubi T, die als Befestigungsschlaufe BS dienen, derart geformt, dass sich das Klebematerial KS auf der Außenseite des jeweiligen Tubus befindet. Der jeweilige Tubus hat bspw. eine Länge von einem halben Meter.
Schritt S2:
   Die zwei schlauchförmigen Tubi T werden in Längsrichtung in paralleler Anordnung auf die Oberseite des durch das Ausgangsmaterial gebildeten Befestigungsträgers BT aufgeklebt, wobei sich auf einer Unterseite des Befestigungsträgers BT ein Klebstoff KS befindet. Das Ausgangsmaterial ist auch der Befestigungsträger BT. Da die beiden Tubi an ihrer Außenseite jeweils Klebematerial aufweisen, kann das Aufkleben der beiden Tubi auf den Befestigungsträger durch Andrücken der Tubi in der geforderten Ausrichtung erfolgen.
Schritt S3:
   In Längsrichtung wird über den mit den Tubi versehenen Befestigungsträger ein weiteres dehnbares Material DMW, welches auf seiner Unterseite ein Klebematerial K aufweist, mit seiner Unterseite auf den Tubi aufgebracht. Das weitere dehnbare Material kann das Ausgangsmaterial sein. Insbesondere wird das weitere dehnbare Material links und rechts der Tubi mit dem Befestigungsträger verklebt.

Durch die Schritte S1 bis S3 entsteht die Vorrichtung V gemäß Figur 6, bei der die Tubi wie ein Sandwich zwischen dem Befestigungsträger und dem weitere dehnbare Material eingeschlossen ist. Figur 6 zeigt einen Querschnitt durch die Vorrichtung und Figur 7 eine Draufsicht auf die Vorrichtung. Je nach Verwendungszweck kann eine Länge der Vorrichtung V konfiguriert werden. Ferner kann die Vorrichtung V in endloser Form produziert werden und durch einen Arzt nach Bedarf in der benötigten Länge zugeschnitten werden. Figur 7 zeigt eine Draufsicht der Vorrichtung mit den beiden Tubi, die zur Führung der beiden Faserbänder ausgebildet sind.

In diesem Ausführungsbeispiel ist auf die Herstellung der Befestigungsmittel BM, welche zur Fixierung der Faserbänder verwendet wird, nicht näher eingegangen worden. Das Befestigungsmittel BM der jeweiligen Befestigungseinheiten BE, die in Figur 6 bzw. 7 nicht abgebildet sind, wird analog zu den Erläuterungen gemäß Figur 3 ausgeführt.

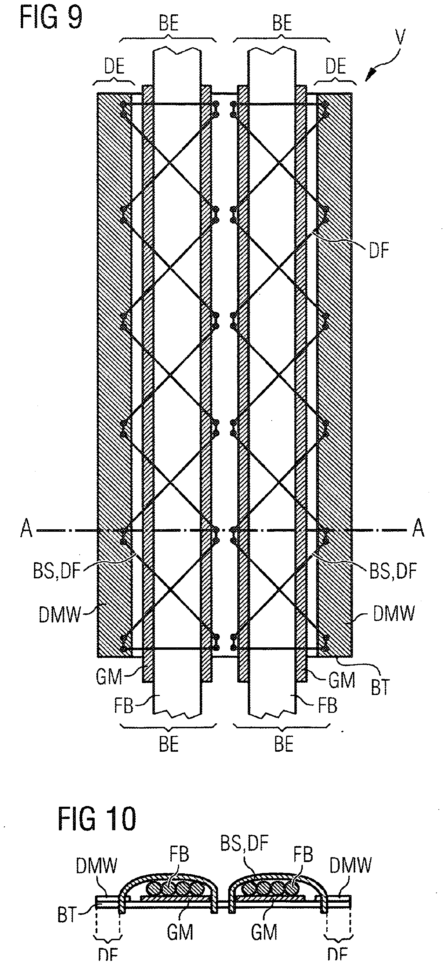

Ein letztes Ausführungsbeispiel wird anhand der Figuren 9 und 10 näher erläutert. Figur 10 zeigt einen Querschnitt im Bereich A-A der Vorrichtung V aus Figur 9.

Die Vorrichtung V gemäß der Figuren 9 und 10 umfasst zwei Befestigungseinheiten BE, wobei diese derart angeordnet sind, dass die in jeweiligen Befestigungseinheiten geführten Faserbänder in Längsrichtung der Faserbänder parallel verlaufen.

Beide Befestigungseinheiten sind auf einem gemeinsamen dehnbaren Befestigungsträger BT, welches auf seiner Unterseite ein Klebmaterial aufweist, mit dem die Vorrichtung auf dem Körperteil aufklebbar ist, angeordnet. Der Befestigungsträger ist insbesondere ein Pflaster z.B. CureTape®. Die Befestigungsschlaufen BS sind in diesem Ausführungsbeispiel aus Dehnungsfäden DF ausbildet, wobei diese Dehnungsfäden unter Anderem im Nähbedarf als elastischer Nähfaden erhältlich sind, wie bspw. von einer Firma Gold-Zack mit einem Namen Strick-Elastic oder eine Gummilitze einer Firma Rieckmann Kurzwaren GmbH. Hierbei sind die Befestigungsschlaufen der jeweiligen Befestigungseinheiten BE aus jeweils zwei sich überkreuzenden Dehnungsfäden gebildet, wobei sich diese überkreuzten Dehnungsfäden in Längsachse der Vorrichtung wiederholt fortsetzen. Zwischen den sich überkreuzenden Dehnungsfäden und dem Befestigungsträger befindet sich das jeweilige Faserband. Zusätzlich kann zwischen Faserband und Befestigungsträger ein zum Gleiten des Faserbands förderndes Gleitmaterial GM, insbesondere ein beidseitig beschichtetes Teflonband, eingebracht sein. Ferner kann auf links und/oder rechts der jeweiligen Faserbänder verlaufenden Bereiche DE des dehnbaren Befestigungsträgers BT ein weiteres dehnbares Material DMW mit seiner mit Klebematerial KS versehenen Unterseite aufgebracht sein. In einer optionalen Weiterbildung können die Dehnungsfäden DF mit dem weiteren dehnbaren Material DMW verbunden sein. Das Befestigungsmittel BM der jeweiligen Befestigungseinheiten BE, die in Figur 9 nicht abgebildet sind, kann analog zu den Erläuterungen gemäß Figur 3 ausgeführt werden.

Es wird noch angemerkt, dass in den Figuren 1 bis 10 eine Beschaltung der Fasern zum Einkoppeln eines jeweiligen Lichtstrahls und zum Erfassen eines gedämpften Lichtstrahls am Ende der jeweiligen Faser nicht dargstellt ist.

### Literaturverweis

[1] US 5,097,252

## Patentansprüche

1. Vorrichtung (V) zum Befestigen zumindest eines Faserbands (FB) auf einem Körperteil (K), wobei das Faserband (FB) zumindest eine Faser (F) umfasst, die als faseroptischer Biegesensor ausgestaltet ist, wobei
a) die Vorrichtung (V) zumindest eine Befestigungseinheit (BE) aufweist,
b) die jeweilige Befestigungseinheit (BE)
b1) ein Befestigungsmittel (BM) zum Anbringen eines Teils des Faserbands (FB) an dem Körperteil (K) und
b2) zumindest eine Befestigungsschlaufe (BS) zum Führen des Faserbands (FB) in einer Achse (x) eines dreidimensionalen Raums, wobei die Befestigungsschlaufe (BS) auf dem Körperteil (K) befestigbar ist,
umfasst, **dadurch gekennzeichnet, dass**
c) die zumindest eine Befestigungsschlaufe (BS) und das Befestigungsmittel (BM) der zumindest einen Befestigungseinheit (BE) auf einem dehnbaren Befestigungsträger (BT), insbesondere einem Pflastermaterial, aufgebrach , wobei der dehnbare Befestigungsträger (BT) mit dem Körperteil (K) durch ein Klebematerial (KS) befestigbar ist.

2. Vorrichtung (V) nach Anspruch 1,
**dadurch gekennzeichnet, dass**
die Vorrichtung (V) eine einzige Befestigungseinheit (BE) mit einem einzigen Befestigungsmittel (BM) und zwei oder mehreren Befestigungsschlaufen (BS) umfasst,
das Körperteil (K) ein Rücken ist,
das Befestigungsmittel (BM) oberhalb der Befestigungsschlaufen (BS), insbesondere im oberen Bereich des Rückens, angeordnet ist,
die Befestigungsschlaufen (BS) untereinander am Rücken angeordnet sind.

3. Vorrichtung (V) nach Anspruch 1,
**dadurch gekennzeichnet, dass**
die Vorrichtung (V) zwei oder mehrere Befestigungseinheiten (BE) umfasst,
jede der Befestigungseinheiten (BE) ein einziges Befestigungsmittel (BM), zumindest eine Befestigungsschlaufe (BS) und ein Faserband (FB) mit zumindest einer Faser(F) umfasst, die Befestigungseinheiten (BE) untereinander am Rücken angeordnet sind.

4. Vorrichtung (V) nach Anspruch 3,
**dadurch gekennzeichnet, dass**
das Befestigungsmittel (BM) einer ersten Befestigungseinheit (BE1) oberhalb einem unterem Ende (UE) des Faserbands (FB) einer, oberhalb der ersten Befestigungseinheit (BE1) angebrachten, zweiten Befestigungseinheit (BE2) angeordnet ist.

5. Vorrichtung (V) nach Anspruch 3 oder 4,
**dadurch gekennzeichnet, dass**
jede Befestigungseinheit (BE) eine sensitive Zone (SZ) für zumindest eine Achse (y) eines dreidimensionalen Raums umfasst.

6. Vorrichtung (V) nach einem der vorhergehenden Ansprüche,
**dadurch gekennzeichnet, dass**
die Befestigungsschlaufen (BS) der zumindest einen Befestigungseinheit (BE) parallel zu einer Wirbelsäule des Rückens oder auf der Wirbelsäule des Rückens anordenbar sind.

7. Vorrichtung nach Anspruch 1,
**dadurch gekennzeichnet, dass**
die Vorrichtung (V) zwei Befestigungseinheiten (BE) umfasst, die zwei Befestigungseinheiten (BE) in Längsrichtung parallel nebeneinander angeordnet sind, insbesondere die eine der zwei Befestigungseinheiten (BE) links und die andere der zwei Befestigungseinheiten (BE) rechts von einer Wirbelsäule eines Patienten anordenbar sind.

8. Vorrichtung nach Anspruch 7,
**dadurch gekennzeichnet, dass**
die jeweilige Befestigungseinheit (BE) eine einzige Befestigungsschlaufe (BS) aufweist, wobei die Befestigungsschlaufe (BS) aus einem in Längsrichtung der Vorrichtung ausgedehnten schlauchförmigen Tubus (T) aus einem dehnbaren Material (TM) mit einem an der Außenseite des Tubus (T) versehenen Klebematerials (KS) gebildet ist,
die derart gebildeten Befestigungsschlaufen (BS) der beiden Befestigungseinheiten (BE) auf dem dehnbaren Befestigungsträger (BT) parallel in Längsrichtung der Vorrichtung (V) angeordnet sind,
eine weiteres in Längsrichtung der Vorrichtung ausgedehntes dehnbares Material (DMW) mit seiner mit Klebematerial (KS) versehnen Unterseite auf den Befestigungsschlaufen (BS) und seitlich der Befestigungsschlaufen (BS) mit dem dehnbaren Befestigungsträger (BT) verbunden ist.

9. Vorrichtung nach Anspruch 7,
**dadurch gekennzeichnet, dass**
die Befestigungsschlaufen (BS) der jeweiligen Befestigungseinheit (BE) aus jeweils zwei sich im Bereich des Faserbands (FB) überkreuzenden Dehnungsfäden (DF) ausgebildet sind.

10. Vorrichtung nach Anspruch 9,
**dadurch gekennzeichnet, dass**
beide Befestigungseinheiten (BE) auf dem einen dehnbaren Befestigungsträger (BT) angebracht sind.

11. Vorrichtung nach Anspruch 10,
**dadurch gekennzeichnet, dass**
der dehnbare Befestigungsträger (BT) lediglich in Längsrichtung der Vorrichtung dehnbar ist.

12. Vorrichtung nach einem der Ansprüche 9 bis 11,
**dadurch gekennzeichnet, dass**
auf links und/oder rechts der jeweiligen Faserbänder (FB) verlaufende Bereiche (BE) des dehnbaren Befestigungsträgers (BT) ein weiteres dehnbares Material (DMW) mit seiner mit Klebematerial (KS) versehnen Unterseite aufgebracht ist.

13. Vorrichtung nach Anspruch 12,
**dadurch gekennzeichnet, dass**
die Dehnungsfäden (DF) mit dem weiteren dehnbaren Material (DMW) verbunden sind.

14. Vorrichtung nach einem der vorhergehenden Ansprüche,
**dadurch gekennzeichnet, dass**
im Bereich der jeweiligen Befestigungsschlaufe (BS) unter eine zum Körper (K) zeigende Seite des jeweiligen Faserbands (FB) ein zum Gleiten des Faserbands (FB) förderndes Gleitmaterial (GM) angeordnet ist.

15. Vorrichtung nach Anspruch 14,
**dadurch gekennzeichnet, dass**
das Gleitmaterial (GM) ein beidseitig beschichtetes Teflonband ist.

16. Verfahren zum Herstellen einer Vorrichtung (V) zum Befestigen von zwei Faserbändern in paralleler Anordnung mittels eines Ausgangsmaterials umfassend ein Trägermaterial (TM) und ein auf einer Unterseite des Trägermaterials (TM) angeordnetes Klebematerial (KS) auf einem Körperteil (K), **gekennzeichnet durch** folgende Schritte:
- aus einem werden zwei schlauchförmige Tubi (T) derart geformt, dass sich das Klebematerial (KS) auf der Außenseite des jeweiligen Tubus befindet.
- die zwei schlauchförmigen Tubi (T) werden in Längsrichtung in paralleler Anordnung auf die Oberseite des **durch** das Ausgangsmaterial gebildeten Befestigungsträgers (BT) aufgeklebt, wobei der Befestigungsträger (BT) zum Anbringen der Vorrichtung (V) auf ein Körperteil (K) ausgestaltet ist.
- In Längsrichtung wird über den mit den zwei schlauchförmigen Tubi (T) versehenen Befestigungsträger ein weiteres dehnbares Material (DMW) in Form des Ausgangsmaterials mit Hilfe des Klebematerial (KS), aufgebracht.

## Claims

1. Device (V) for fixing at least one fibrous strip (FB) to a body part (K), with the fibrous strip (FB) comprising at least one fibre (F) which is embodied as a fibre optic bending sensor, wherein
a) the device (V) having at least one fixing unit (BE) comprises,
b) the respective fixing unit (BE)
b1) a fixing means (BM) for attaching a part of the fibrous strip (FB) to the body part (K) and
b2) at least one fixing loop (BS) for guiding the fibrous strip (FB) within an axis (x) of a three-dimensional space, with the fixing loop (BS) able to be fixed to the body part (K),
**characterised in that**
the at least one fixing loop (BS) and/or the fixing means (BM) of the at least one fixing unit (BE) are attached to an expandable fixing support (BT), especially a sticking plaster material, with the expandable fixing support (BT) able to be fixed to the body part (K) by an adhesive material (KS).

2. Device (V) according to claim 1,
**characterised in that**
the device (V) comprises a single fixing unit (BE) with a single fixing means (BM) and two or more fixing loops (BS), the body part (K) is a back,
the fixing means (BM) is arranged above the fixing loop (BS), especially in the upper area of the back,
the fixing loops (BS) are arranged below one another on the back.

3. Device (V) according to claim 1,
**characterised in that**
the device (V) comprises two or more fixing units (BE),
each of the fixing units (BE) comprises a single fixing means (BM), at least one fixing loop (BS) and a fibrous strip (FB) with at least one fibre (F),
the fixing units (BE) are arranged below one another on the back.

4. Device (V) according to claim 3,
**characterised in that**
the fixing means (BM) of a first fixing unit (BE1) is arranged above a lower end (UE) of the fibrous strip (FB) of a second fixing unit (BE2) attached above the first fixing unit (BE1).

5. Device (V) according to claim 3 or 4,
**characterised in that**
each fixing unit (BE) comprises a sensitive zone (SZ) for at least one axis (y) of a three-dimensional space.

6. Device (V) according to one of the preceding claims,
**characterised in that**
the fixing loops (BS) of the at least one fixing unit (BE) are able to be arranged in parallel to a spinal column of the back or on the spinal column of the back.

7. Device according to claim 1,
**characterised in that**
the device (V) comprises two fixing units (BE), the two fixing units (BE) are arranged in a longitudinal direction in parallel next to one another, especially the one of the two fixing units (BE) is able to be arranged to the left and the other of the two fixing units (BE) to the right of a spinal column of a patient.

8. Device according to claim 7,
**characterised in that**
the respective fixing unit (BE) has a single fixing loop (BS), with the fixing loop (BS) being embodied from an extended loop-shaped tube (T) in the longitudinal direction of the device made of an expandable material (TM) provided with an adhesive material (KS) on the outer side of the tube (T),
the fixing loops (BS) of the two fixing units (BE) embodied in this way are arranged on an expandable fixing support (BT) in parallel in the longitudinal direction of the device (V),
a further expandable material (DMW) expanded in the longitudinal direction of the device with its underside coated with adhesive material (KS) is connected to the fixing loops (BS) and to the side of the fixing loops (BS) to the expandable fixing support (BT).

9. Device according to claim 7,
**characterised in that**
the fixing loops (BS) of the respective fixing unit (BE) are each embodied from two expansion fibres (DF) crossing over each other in the area of the fibrous strip (FB).

10. Device according to claim 9,
**characterised in that**
the two fixing units (BE) are attached to the one expandable fixing support (BT).

11. Device according to claim 10,
**characterised in that**
the expandable fixing support (BT) is merely expandable in the longitudinal direction of the device.

12. Device according to one of claims 9 to 11,
**characterised in that**,
attached to areas (BE) of the expandable fixing support (BT) running on the left and/or right of the respective fibrous strips (FB) is a further expandable material (DMW) with its underside coated with adhesive material (KS).

13. Device according to claim 12,
**characterised in that**,
the expansion fibres (DF) are connected to the further expandable material (DMW).

14. Device according to one of the preceding claims,
**characterised in that**,
arranged in the area of the respective fixing loop (BS) below a side of the respective fibrous strip (FB) pointing towards the body (K) is a lubricant material (GM) to promote the sliding of the fibrous strip (FB).

15. Device according to claim 14,
**characterised in that**
the lubricant material (GM) is a Teflon tape coated on both sides.

16. Method for production of a device (V) for fixing two fibrous strips in a parallel arrangement by means of a base material comprising a support material (TM) and an adhesive material (KS) arranged on an underside of the support material (TM) to a body part (K),
**characterised by** the following steps:
- two loop-shaped tubes (T) are formed from one such that the adhesive material (KS) is located on the outer side of the respective tube.
- the two loop-shaped tubes (T) are glued in the longitudinal direction in a parallel arrangement to the upper side of the fixing support (BT) formed by the base material, with the fixing support (BT) being embodied for attaching the device (V) to a body part (K).
- in the longitudinal direction a further expandable material (DMW) in the form of the base material is attached with the aid of the adhesive material (KS), above the fixing support provided with the two loop-shaped tubes (T).

## Revendications

1. Dispositif (V) pour fixer au moins une bande de fibres (FB) sur une partie corporelle (K), la bande de fibres (FB) comprenant au moins une fibre (F) qui est réalisée en tant que détecteur de flexion à fibre optique,
a) le dispositif (V) comprenant au moins une unité de fixation (BE),
b) l'unité de fixation respective (BE) comprenant
b1) un moyen de fixation(BM) pour appliquer une partie de la bande de fibres (FB) sur la partie corporelle (K) et
b2) au moins une boucle de fixation (BS) pour guider la bande de fibres (FB) dans un axe (x) d'un espace tridimensionnel, la boucle de fixation (BS) pouvant être fixée sur la partie corporelle (K),
**caractérisé en ce que**
c) l'au moins une boucle de fixation (BS) et le moyen de fixation (BM) de l'au moins une unité de fixation (BE) sont placés sur un support de fixation extensible (BT), notamment un matériau de type pansement, le support de fixation extensible (BT) pouvant être fixé à la partie corporelle (K) par une matière adhésive (KS).

2. Dispositif (V) selon la revendication 1, **caractérisé en ce que**
le dispositif (V) comprend une seule unité de fixation (BE) avec un seul moyen de fixation (BM) et deux ou plusieurs boucles de fixation (BS),
la partie corporelle (K) est un dos,
le moyen de fixation (BM) est situé au-dessus des boucles de fixation (BS), notamment dans la zone supérieure du dos,
les boucles de fixation (BS) sont disposées les unes en dessous des autres sur le dos.

3. Dispositif (V) selon la revendication 1, **caractérisé en ce que**
le dispositif (V) comprend deux ou plusieurs unités de fixation (BE),
chacune des unités de fixation (BE) comprend un seul moyen de fixation (BM), au moins une boucle de fixation (BS) et une bande de fibres (FB) avec au moins une fibre (F),
les unités de fixation (BE) sont disposées les unes en dessous des autres sur le dos.

4. Dispositif (V) selon la revendication 3, **caractérisé en ce que**
le moyen de fixation (BM) d'une première unité de fixation (BE1) est situé au-dessus d'une extrémité inférieure (UE) de la bande de fibres (FB) d'une seconde unité de fixation (BE2) placée au-dessus de la première unité de fixation (BE1).

5. Dispositif (V) selon la revendication 3 ou 4, **caractérisé en ce que**
chaque unité de fixation (BE) comprend une zone sensible (SZ) pour au moins un axe (y) d'un espace tridimensionnel.

6. Dispositif (V) selon l'une des revendications précédentes, **caractérisé en ce que**
les boucles de fixation (BS) de l'au moins une unité de fixation (BE) peuvent être disposées parallèlement à la colonne vertébrale du dos ou sur la colonne vertébrale du dos.

7. Dispositif selon la revendication 1, **caractérisé en ce que**
le dispositif (V) comprend deux unités de fixation (BE),
les deux unités de fixation (BE) sont disposées parallèlement l'une à côté de l'autre dans la direction longitudinale, en particulier, l'une des deux unités de fixation (BE) peut être placée à gauche de la colonne vertébrale d'un patient tandis que l'autre unité de fixation (BE) peut être placée à droite de sa colonne vertébrale.

8. Dispositif selon la revendication 7, **caractérisé en ce que**
l'unité de fixation respective (BE) comprend une seule boucle de fixation (BS), la boucle de fixation (BS) étant formée par un tube (T) en forme de tuyau formé d'un matériau extensible (TM) s'étendant dans la direction longitudinale du dispositif, pourvu d'une matière adhésive (KS) sur le côté extérieur du tube (T),
les boucles de fixation (BS) ainsi formées des deux unités de fixation (BE) sont disposées de manière parallèle sur le support de fixation extensible (BT) dans la direction longitudinale du dispositif (V),
un autre matériau extensible (DMW) s'étendant dans la direction longitudinale du dispositif est relié au support de fixation extensible (BT), sur les côtés des boucles de fixation (BS), avec son côté inférieur pourvu de matière adhésive (KS) posé sur les boucles fixation (BS).

9. Dispositif selon la revendication 7, **caractérisé en ce que**
les boucles de fixation (BS) de l'unité de fixation respective (BE) sont formées à chaque fois par deux fils élastiques (DF) se croisant dans la zone de la bande de fibres (FB).

10. Dispositif selon la revendication 9, **caractérisé en ce que**
les deux unités de fixation (BE) sont placées sur le support de fixation extensible (BT).

11. Dispositif selon la revendication 10, **caractérisé en ce que**
le support de fixation extensible (BT) est extensible uniquement dans la direction longitudinale du dispositif.

12. Dispositif selon l'une des revendications 9 à 11, **caractérisé en ce que**
un autre matériau extensible (DMW) est posé, avec son côté inférieur pourvu d'une matière adhésive (KS), sur les zones (BE) du support de fixation extensible (BT) s'étendant à gauche et/ou à droite des bandes de fibres respectives (FB).

13. Dispositif selon la revendication 12, **caractérisé en ce que**
les fils élastiques (DF) sont reliés à l'autre matériau extensible (DMW).

14. Dispositif selon l'une des revendications précédentes, **caractérisé en ce qu'**un matériau glissant (GM) favorisant le glissement de la bande de fibres (FB) est situé dans la zone de la boucle de fixation respective (BS) sous un côté de la bande de fibres respective (FB) dirigé vers le corps (K).

15. Dispositif selon la revendication 14, **caractérisé en ce que**
le matériau glissant (GM) est une bande téflon à revêtement sur deux faces.

16. Procédé de fabrication d'un dispositif (V) pour la fixation sur une partie corporelle (K) de deux bandes de fibres agencées parallèlement au moyen d'un matériau de départ comprenant un matériau de support (TM) et une matière adhésive (KS) située sur un côté inférieur du matériau de support (TM),
**caractérisé par** les étapes suivantes :
- à partir d'un matériau de départ, on forme deux tubes (T) en forme de tuyau de telle sorte que la matière adhésive (KS) se trouve sur le côté extérieur du tube respectif,
- les deux tubes (T) en forme de tuyau sont collés sur le côté supérieur du support de fixation (BT) formé par le matériau de départ, parallèlement l'un à l'autre dans la direction longitudinale, le support de fixation (BT) étant exécuté pour appliquer le dispositif (V) sur une partie corporelle (K),
- un autre matériau extensible (DMW) ayant la forme du matériau de départ est appliqué dans la direction longitudinale, grâce à la matière adhésive (KS), sur le support de fixation pourvu des deux tubes (T) en forme de tuyau.
